(19) Europäisches Patentamt
European Patent Office
Office européen des brevets

(11) Veröffentlichungsnummer: **0 144 284**
**A1**

(12) # EUROPÄISCHE PATENTANMELDUNG

(21) Anmeldenummer: **84810511.0**

(22) Anmeldetag: **22.10.84**

(51) Int. Cl.⁴: **D 21 D 3/00**
D 21 H 3/02, C 07 C 143/83
C 07 C 143/833

(30) Priorität: **27.10.83 CH 5819/83**

(43) Veröffentlichungstag der Anmeldung:
**12.06.85 Patentblatt 85/24**

(84) Benannte Vertragsstaaten:
**AT BE CH DE FR GB IT LI NL SE**

(71) Anmelder: **CIBA-GEIGY AG**
**Postfach**
**CH-4002 Basel(CH)**

(72) Erfinder: **Bernheim, Michael, Dr.**
**Angensteinerweg 15**
**CH-4144 Arlesheim(CH)**

(72) Erfinder: **Meindl, Hubert, Dr.**
**Kornfeldstrasse 77**
**CH-4125 Riehen(CH)**

(72) Erfinder: **Rohringer, Peter**
**Sechsjuchartenstrasse 1**
**CH-4124 Schönenbuch(CH)**

(72) Erfinder: **Wegmüller, Hans, Dr.**
**Kornfeldstrasse 18**
**CH-4125 Riehen(CH)**

(72) Erfinder: **Werthemann, Dieter, Dr.**
**Scherkesselweg 15**
**CH-4052 Basel(CH)**

(54) **Verfahren zum Leimen von Papier mit anionischen, hydrophoben Leimungsmitteln und kationischen Retentionsmitteln.**

(57) Leimungsmittel, welche neue Verbindungen darstellen und mindestens eine anionische oder acide, gegebenenfalls in Salzform vorliegende Sulfonylcarbonylimidgruppe und mindestens zwei hydrophobe Substituenten aufweisen, wobei mindestens zwei hydrophobe Substituenten miteinander über ein Brückenglied der Formel $-CO-NH-A_1-(SO_2-NH-CO)_t$ verknüpft sind, wobei $A_1$ einen zwei- oder dreiwertigen, aliphatischen, cycloaliphatischen oder aromatischen Rest mit höchstens 10 Kohlenstoffatomen darstellt und t 1 oder 2 ist, eignen sich besonders gut dazu, zusammen mit handelsüblichen Retentionsmitteln in einem Verfahren zum Leimen von Papier oder Karton eingesetzt zu werden.

EP 0 144 284 A1

0144284

CIBA-GEIGY AG
Basel (Schweiz)

1-14631/+

# Verfahren zum Leimen von Papier mit anionischen, hydrophoben Leimungsmitteln und kationischen Retentionsmitteln

Der vorliegenden Erfindung liegt die Aufgabe zu Grunde, dem Papierhersteller leicht zugängliche und auf einfache Art erhältliche Leimungsmittel zur Verfügung zu stellen, die unter Mitverwendung von herkömmlichen, kationischen Retentionsmitteln in neuartiger Kombination geeignet sind, eine gute Leimung bei der Papierherstellung aus Faserstoffsuspensionen zu bewirken.

Diese Aufgabe wird erfindungsgemäss so gelöst, dass bei der Papierherstellung unter Mitverwendung von polymeren, kationischen Retentionsmitteln, Leimungsmittel eingesetzt werden, die mindestens zwei langkettige, hydrophobe Substituenten und mindestens eine anionische oder acide (saure), gegebenenfalls in Salzform vorliegende Gruppe aufweisen, wobei die hydrophoben Substituenten über ein oder mehrere Brückenglieder verbunden sind, die mindestens 1 Kohlenstoffatom und mindestens 2 Heteroatome in der Hauptkette enthalten.

Gegenstand der vorliegenden Erfindung ist somit ein Verfahren zum Leimen von Papier oder Karton, das dadurch gekennzeichnet ist, dass man zu wässrigen, cellulosehaltigen, gegebenenfalls füllmittelhaltigen Faserstoffsuspensionen mindestens

(A) ein Leimungsmittel, welches 1 oder 2 anionische oder acide, gegebenenfalls in Salzform vorliegende Sulfonylcarbonylimidgruppen ($-SO_2-NH-CO-$) und 2 bis 6 hydrophobe Substituenten mit jeweils mindestens 5 Kohlenstoffatomen enthält, wobei mindestens einer der hydrophoben Substituenten mindestens 8 Kohlenstoffatome aufweist und mindestens

zwei der hydrophoben Substituenten miteinander über ein Brückenglied verknüpft sind, das der Formel

(1) $-CO-NH-A_1-(SO_2-NH-CO)_t-$

entspricht, worin $A_1$ einen zwei- oder dreiwertigen aliphatischen, cycloaliphatischen oder aromatischen Rest mit höchstens 10 Kohlenstoffatomen und t 1 oder 2 bedeuten, und

(B) ein polymeres, kationisches Retentionsmittel

in beliebiger Reihenfolge oder gleichzeitig hinzugibt.

Weitere Erfindungsgegenstände bilden

- die wässrigen Zusammensetzungen zur Durchführung des Papierleimungsverfahrens, die, sofern das Leimungsmittel (A) und das Retentionsmittel (B) in beliebiger Reihenfolge zur Faserstoffsuspension separat gegeben werden, neben fakultativen üblichen Zusätzen das Leimungsmittel (A) allein, das mindestens teilweise in Form von Salzen vorliegt, oder, sofern das Leimungsmittel (A) und das Retentionsmittel (B) der Faserstoffsuspension gleichzeitig zugegeben werden, sowohl das gegebenenfalls mindestens teilweise in Salzform vorliegende Leimungsmittel (A) als auch das Retentionsmittel (B) neben fakultativen, üblichen Zusätzen enthalten,
- das (der) nach dem erfindungsgemässen Verfahren geleimte Papier oder Karton und
- die Verwendung des Leimungsmittels (A) der angegebenen Art zum Leimen von Papier oder Karton.

Die angegebenen Leimungsmittel (A) stellen neue Verbindungen dar, welche zusammen mit dem Verfahren zu deren Herstellung ebenfalls weitere Gegenstände der vorliegenden Erfindung bilden.

Als wesentliches Merkmal weisen die erfindungsgemässen Leimungsmittel (A) im allgemeinen 1 oder 2 potentiell anionische Gruppen

auf, die in der Regel als acide Sulfonylcarbonylimidgruppen ($-SO_2-NH-CO-$) vorliegen. Leimungsmittel, die nur eine solche potentiell anionische Gruppe aufweisen, sind bevorzugt. Diese potentiell anionischen Gruppen können in wässrigem Medium bei den üblicherweise bei der Papierherstellung vorliegenden pH-Werten der Faserstoffsuspensionen Anionen bilden. Unter den genannten Bedingungen können andererseits auch die kationischen Retentionsmittel (B) Kationen bilden. Die Fähigkeit der Leimungsmittel und der Retentionsmittel, bei den Bedingungen der Papierherstellung Anionen bzw. Kationen zu bilden, kann auch als anionaktiv bzw. kationaktiv bezeichnet werden. Somit können die anionischen Leimungsmittel und die kationischen Retentionsmittel auch anionaktive Leimungsmittel bzw. kationaktive Retentionsmittel genannt werden.

Als weiteres kennzeichnendes Merkmal weisen die Leimungsmittel (A) 2 bis 6, vorzugsweise 2 bis 4 hydrophobe Substituenten mit mindestens 5, vor allem 6 bis 22, vorzugsweise 8 bis 22, insbesondere 16 bis 20 Kohlenstoffatomen, z.B. $C_5-C_{12}$-Cycloalkyl- oder $C_6-C_{10}$-Aryl-, -Alkaryl- oder -Aralkylreste auf. Bevorzugte hydrophobe Substituenten enthalten indessen unsubstituierte oder mit $C_1-C_4$-Alkyl substituierte Phenylreste, insbesondere Alkyl- oder Alkenylreste, welche sich in der Regel von ungesättigten oder gesättigten Fettsäuren, Fettalkoholen oder Fettaminen mit mindestens 6, vorzugsweise 8 bis 22, insbesondere 16 bis 20 Kohlenstoffatomen ableiten. Die hydrophoben Substituenten enthalten einen oder mehrere Reste, die nur aus Kohlenstoff und Wasserstoff bestehen, z.B. Cycloalkyl-, Aryl-, Alkaryl-, Aralkyl-, Alkyl- oder Alkenyl-Reste der angegebenen Art, und zusätzlich einen zwei- oder mehrwertigen Rest, über welchem die hydrophoben Substituenten an das Brückenglied der Formel (1) gebunden sind. Diese zusätzlichen zwei- oder mehrwertigen Reste bestehen aus einem zweiwertigen Sauerstoffatom, einem sekundären oder tertiären Aminrest, einem Alkylendiaminrest oder einem Polyalkylenpolyaminrest.

Die Verknüpfungsart, mit welcher die hydrophoben Substituenten miteinander verbunden sind, bildet ein weiteres Kennzeichen der Leimungsmittel (A). Die Brückenglieder, über welche mindestens zwei der hydrophoben Substituenten verknüpft sind, entsprechen nämlich der vorstehend angegebenen Formel (1) und tragen vorzugsweise 1 bis 13, insbesondere 1 bis 10 Kohlenstoffatome und mindestens 2 Stickstoff- und 1 Schwefelatom als Heteroatome in der Hauptkette, vorzugsweise 1 oder 2 Schwefel- und 2 oder 3 Stickstoffatome.

Sofern t in Formel (1) 2 ist, ist der Rest $A_1$ des Brückenglieds dreiwertig. Zweiwertige Reste für $A_1$ bei Brückengliedern der Formel (1), worin t 1 ist, sind indessen bevorzugt. Als Definition von $A_1$ kommen zwei- oder dreiwertige, verzweigte oder vorzugsweise geradkettige Alkylreste mit 2 bis 10, insbesondere 2 bis 6 Kohlenstoffatomen, zwei- oder dreiwertige Cyclopentyl- vorzugsweise Cyclohexylreste, mit Aethyl oder vorzugsweise Methyl substituierte, insbesondere unsubstituierte zwei- oder dreiwertige Phenylreste und zwei- oder dreiwertige Dihydronaphthalin-, Tetrahydronaphthalin-, Decalin-, vorzugsweise Naphthalinreste in Betracht.

In ihrer bevorzugten Ausführungsart enthalten demgemäss bevorzugte Leimungsmittel eine acide Sulfonylcarbonylimidgruppe und 2 bis 4 hydrophobe Substituenten mit jeweils 8 bis 22 Kohlenstoffatomen der vorstehend angegebenen Art, wobei mindestens zwei der hydrophoben Substituenten über ein Verbindungsglied der Formel

(2) $-CO-NH-A_2-SO_2-NH-CO-$,

worin $A_2$ Cycloalkylen, Naphthylen, vor allem durch Methyl substituiertes Phenylen, vorzugsweise Toluylen und Xylylen, insbesondere unsubstituiertes Phenylen oder Alkylen mit 2 bis 6 Kohlenstoffatomen, bedeutet, verknüpft sind.

Als Bedeutungen von $A_2$ in Formel (2) stehen Isopropylen, Propylen, Xylylen, Toluylen, vor allem Aethylen und m-Phenylen und insbesondere p-Phenylen im Vordergrund des Interesses.

Von besonderer Bedeutung als Leimungsmittel (A) sind vor allem solche, die durch chemische Umsetzung von mindestens

(a) 1 Mol einer Verbindung der Formel

(3) $O=C=N-A_1-(SO_2-N=C=O)_t$ ,

worin $A_1$ und t die angegebenen Bedeutungen haben, mit

(b) t+1 Mol eines Fettalkohols, eines primären oder sekundären Fettamins und/oder eines Kondensationsproduktes aus einer Fettsäure und einem Alkylendiamin oder Polyalkylenpolyamin, wobei das Kondensationsprodukt mindestens eine freie Aminogruppe aufweist,

erhältlich sind.

Die Di- oder Monosulfonylisocyanate der Formel (3) als Komponente (a), aus welchen die Leimungsmittel (A) erhältlich sind, sind z.B. aus den US Patentschriften 3 454 606 und 3 330 848 an sich bekannt und werden nach bekannten Methoden hergestellt. Monosulfonylisocyanate sind den Disulfonylisocyanaten gegenüber als Komponente (a) bevorzugt und entsprechen der Formel

(4) $O=C=N-A_2-SO_2-N=C=O$ ,

worin $A_2$ die angegebenen Bedeutungen hat.

Als Komponente (b), aus welchen das Leimungsmittel (A) erhältlich ist, kommen vor allem ungesättigte, vorzugsweise gesättigte aliphatische Fettalkohole mit 6 bis 22, vorzugsweise 8 bis 22, insbesondere 16 bis 20 Kohlenstoffatomen in Betracht. Sofern die Komponente (b) ein Fettamin ist, so handelt es sich im allgemeinen um Mono- oder Dialkylamine oder Mono- oder Dialkenylamine mit jeweils 6 bis 22, vorzugsweise 8 bis 22, insbesondere 16 bis 20 Kohlenstoffatomen im Alkyl- oder Alkenylrest. Die Mono- oder Dialkylamine sind den Mono- oder Dialkenylaminen gegenüber als Fettamine bevorzugt. Als spezifische Vertreter von $C_{16}$-$C_{20}$-Fettalkoholen und von Mono- oder Dialkylaminen mit $C_{16}$-$C_{20}$-Alkylresten für die Komponente (b) seien ihrer guten Zugänglichkeit wegen Hexadecanol, Octadecanol, Oleylalkohol, Octadecylamin und Dioctadecylamin erwähnt. Auch technische Gemische der Fettalkohole bzw. der Fettamine der angegebenen Art kommen in Betracht.

Die Fettalkohole und Fettamine der vorstehend angegebenen Art als Komponente (b) leiten sich strukturell von ungesättigten oder gesättigten $C_6$-$C_{22}$, vorzugsweise $C_8$-$C_{22}$-, insbesondere $C_{16}$-$C_{20}$-Fettsäuren ab. Bei diesen handelt es sich z.B. um Capron-, vorzugsweise Capryl-, Caprin-, Laurin-, Myristin- oder Myristolein-, Palmitolein-, Elaeostearin-, Clupanodonsäure, insbesondere Oel-, Elaidin-, Eruka-, Linol- und Linolensäure. Hierbei kommt der Palmitin-, Stearin-, Oel- und Behensäure eine besondere Bedeutung zu, wobei Palmitin- und vor allem Stearinsäure im Vordergrund des Interesses stehen. Auch Fettalkohole und Fettamine, die sich von technischen, gut zugänglichen Gemischen der soeben erwähnten Säuren ableiten, kommen in Betracht. Synthetische Fettalkohole, die z.B. durch Oxosynthese hergestellt werden, werden von der angegebenen Definition auch umfasst.

Ist die Komponente (b), aus welcher das Leimungsmittel (A) in einer weiteren Ausführungsart erhältlich ist, ein Kondensationsprodukt aus Fettsäuren und Alkylendiaminen oder Polyalkylenpolyaminen, so weist das Kondensationsprodukt wie bereits angedeutet stets eine freie Aminogruppe auf. Die Fettsäuren oder technischen Fettsäuregemische der vorstehend angegebenen Art sind als Ausgangskomponente zur Herstellung solcher Kondensationsprodukte bevorzugt.

Die Alkylendiamine oder Polyalkylenpolyamine als Ausgangskomponente zur Herstellung der Kondensationsprodukte entsprechen in ihrer bevorzugten Ausführungsform der Formel

(5) $H_2N-D_1-(NH-D_2)_{n-1}-NH_2$,

worin $D_1$ und $D_2$ voneinander verschieden oder vorzugsweise gleich sind und je Propylen oder insbesondere Aethylen und n eine ganze Zahl von

1 bis 5, vorzugsweise 1, 2 oder 3, insbesondere 2, bedeuten. Als spezifische Vertreter seien z.B. Tetraäthylenpentamin, Triäthylentetramin, vor allem Aethylendiamin und insbesondere Diäthylentriamin erwähnt.

Da das Kondensationsprodukt stets mindestens eine freie Aminogruppe aufweisen muss, werden pro Mol Alkylendiamin oder Polyalkylenpolyamin der Formel (5) m Mol Fettsäure oder Fettsäuregemisch der angegebenen Art eingesetzt, wobei m eine ganze Zahl von 1 bis n bedeutet. So werden z.B. 1 Mol Fettsäure pro Mol Alkylendiamin, 1 oder 2 Mol Fettsäure pro Mol Dialkylentriamin und 1,2,3 oder 4 Mol Fettsäure pro Mol Tetraäthylenpentamin eingesetzt. Somit entsprechen Kondensationsprodukte in ihrer bevorzugten Ausführungsform als Komponente (b), aus welcher das Leimungsmittel (A) erhältlich ist, der wahrscheinlichen Formel

$$(6)\quad \left\{ \left[ \overset{|}{H}N-D_1-(\overset{|}{N}-D_2)_{n-1}-\overset{|}{N}H \right] \quad \begin{matrix} \left[ -\overset{\overset{O}{\parallel}}{C}-R_1 \right]_m \\ \left[ -H \right]_{n-m+1} \end{matrix} \right\} ,$$

worin $R_1$ Alkyl oder Alkenyl mit 6 bis 22, vorzugsweise 8 bis 22, insbesondere 16 bis 20 Kohlenstoffatomen bedeutet und $D_1$, $D_2$, m und n die angegebenen Bedeutungen haben.

In der Regel werden als Komponente (b), aus welcher das Leimungsmittel (A) erhältlich ist, entweder Fettalkohole oder Fettamine oder Kondensationsprodukte aus Fettsäuren und Alkylendiaminen oder Polyalkylenpolyaminen der angegebenen Art eingesetzt. Gemische aus Fettalkoholen und Fettaminen, aus Fettalkoholen und Kondensationsprodukten der angegebenen Art oder aus Fettaminen und Kondensationsprodukten der angegebenen Art oder sogar aus Fettalkoholen, Fettaminen und Kondensationsprodukten der angegebenen Art kommen jedoch auch in Frage.

Die an sich neuen Verbindungen, die als erfindungsgemässe Leimungsmittel (A) verwendbar sind, entsprechen der wahrscheinlichen Formel

(7) 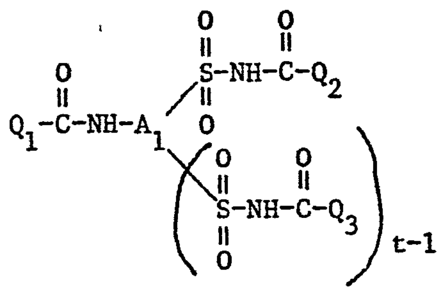 ,

worin $Q_1$, $Q_2$ und $Q_3$ voneinander verschieden oder vorzugsweise gleich sind und je $-O-R_1$, $-NH-R_1$, 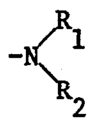 oder

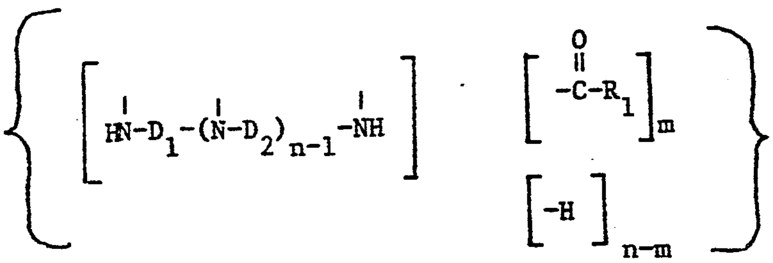 ,

$R_1$ und $R_2$ voneinander verschieden oder vorzugsweise gleich sind und je Alkyl oder Alkenyl mit 6 bis 22, vorzugsweise 8 bis 22, insbesondere 16 bis 20 Kohlenstoffatomen und $A_1$, $D_1$, $D_2$, n, m und t die angegebenen Bedeutungen haben, oder insbesondere der wahrscheinlichen Formel

(8) 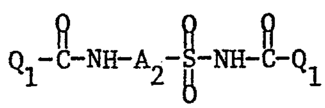 ,

worin

$Q_1$ $-OR_1$, $-NH-R_1$, 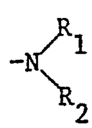 oder

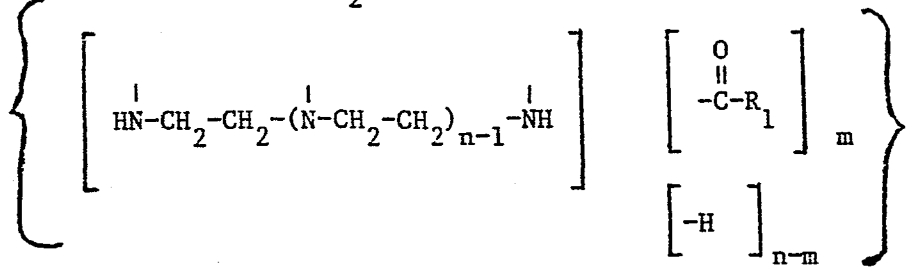 ,

und $A_2$, $R_1$, $R_2$, n und m die angegebenen Bedeutungen haben.

Je nach Bedeutung von $Q_1$, $Q_2$ und $Q_3$ in Formel (7) entsprechen die neuen Verbindungen, bei welchen in ihrer bevorzugten Ausführungsart $Q_1$, $Q_2$ und $Q_3$ gleich sind, entweder der Formel

(9) 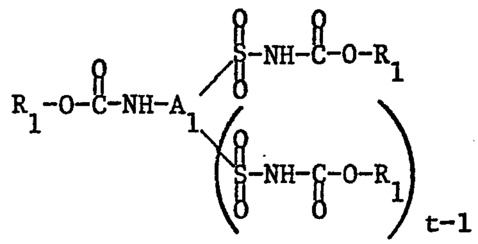 ,

insbesondere

(10) 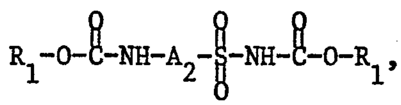 ,

der Formel

(11) 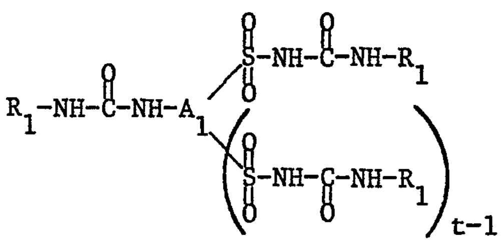 ,

insbesondere

(12) 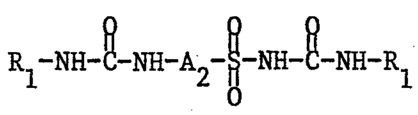 ,

der Formel

(13) 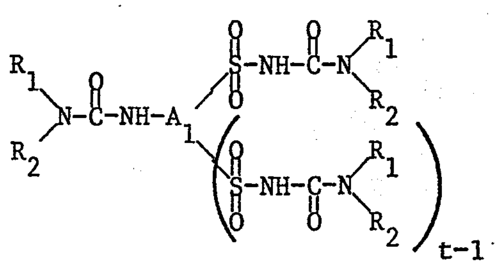 ,

insbesondere

(14) 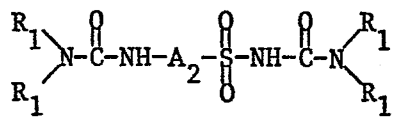

oder der wahrscheinlichen Formel

(15) 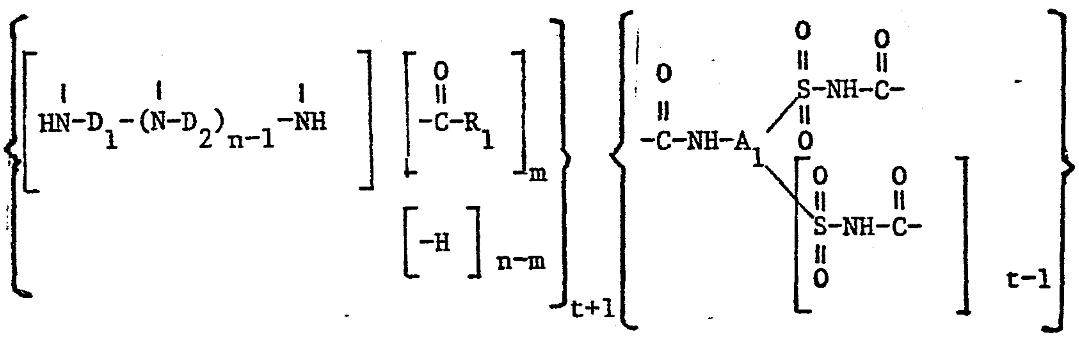 ,

insbesondere

(16) 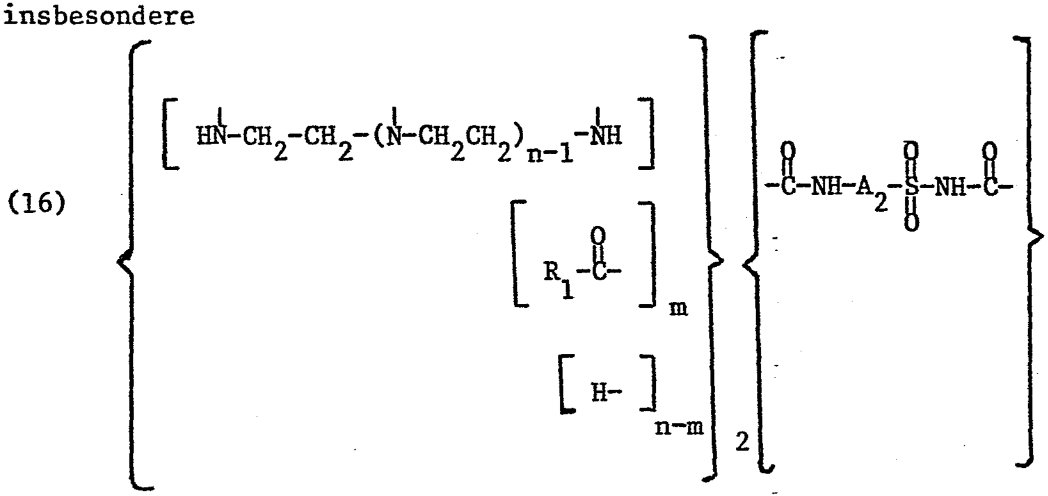 ,

wobei $A_1$, $A_2$, $D_1$, $D_2$, $R_1$, $R_2$, n, m und t die angegebenen Bedeutungen haben.

Als spezifische Vertreter von neuen Verbindungen, die erfindungsgemäss als Leimungsmittel (A) verwendbar sind und im Vordergrund des Interesses stehen, seien u.a. solche der Formel

(17) 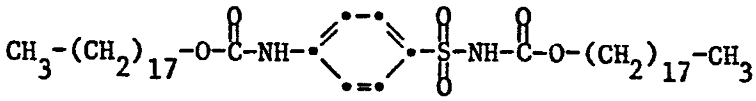 ,

(18) 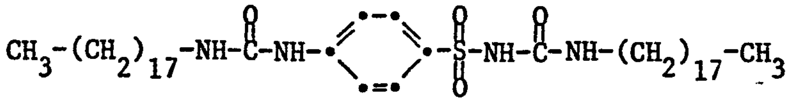 ,

(19) 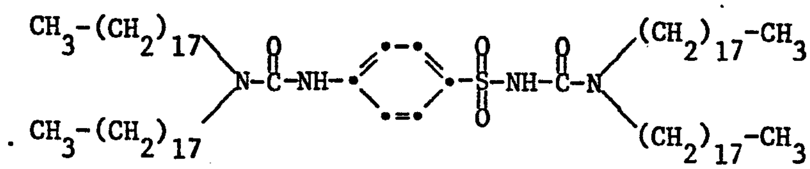

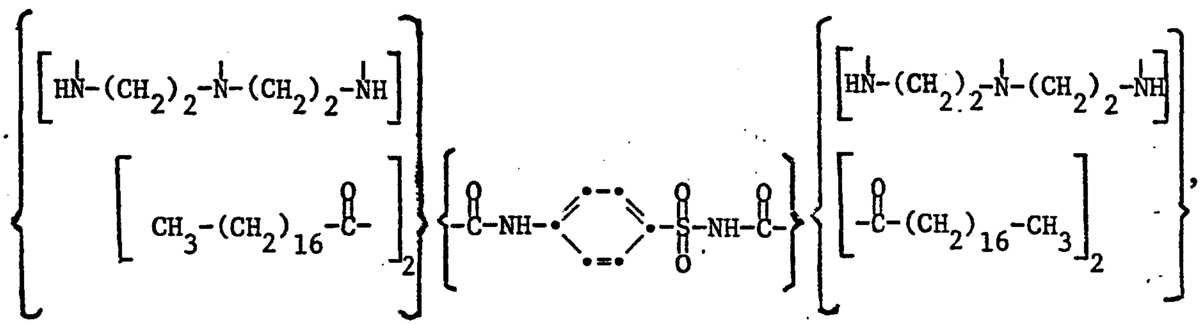 ,

(20)

(21) 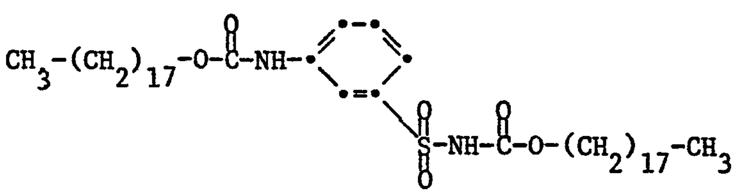

und

(22) 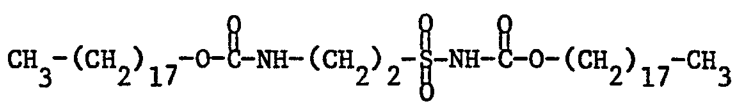

erwähnt.

Das Verfahren zur Herstellung der neuen Verbindungen der Formel (7) zeichnet sich dadurch aus, dass man nach an sich bekannten Methoden

1 Mol der Verbindung der Formel (3)
mit t+1 Mol eines Fettalkohols der Formel

(23) $R_1$-OH ,

mit t+1 Mol eines primären oder sekundären Fettamins der Formel

(24) $R_1$-$NH_2$ oder

(25) $R_1$-NH-$R_2$ , oder

mit t+1 Mol eines Kondensationsproduktes der Formel (6) aus m Mol einer Fettsäure und 1 Mol eines Alkylendiamins oder Polyalkylenpolyamins, wobei $A_1$, $D_1$, $D_2$, $R_1$, $R_2$, m, n und t die angegebenen Bedeutungen haben, umsetzt. Obwohl vorzugsweise jeweils t+1 Mol einer der Verbindungen (23), (24), (25) oder (6) mit 1 Mol der Verbindungen der Formel (31) umgesetzt werden, ist es auch möglich t+1 Mol eines Gemisches der Verbindungen (23), (24), (25) und/oder (6) einzusetzen.

Die Umsetzungen der Komponente (a) mit der Komponente (b) werden vorzugsweise bei Temperaturen von höchstens 90°C, vorzugsweise -10 bis +60°C, insbesondere 10 bis 40°C im allgemeinen in Gegenwart eines Lösungsmittels, das gegenüber den Ausgangskomponenten und den Endprodukten inert sein muss, durchgeführt. Als Beispiele möglicher Lösungsmittel seien Aether, wie Diäthyläther, Diisopropyläther oder gegebenenfalls halogenierte Kohlenwasserstoffe wie etwa Dichloräthan, Tetrachlorkohlenstoff, ferner Benzol, Toluol, Chlorbenzol, o-, m- und p-Xylol, ein technisches Xylolgemisch oder auch Gemische der erwähnten Kohlenwasserstoffe genannt. Im Hinblick auf die grosse Reaktivität der Komponente (a) mit den Komponenten (b) empfiehlt es sich, die Komponente (b) zur Komponente (a) z.B. innerhalb 1/4 bis 3 Stunden, insbesondere 1/2 bis 1 1/2 Stunden zu geben, wobei das Reaktionsgemisch in der Regel gekühlt werden muss.

Vor ihrem Einsatz als Komponente (A) im erfindungsgemässen Papierleimungsverfahren brauchen die Leimungsmittel nach erfolgter Herstellung im allgemeinen durch z.B. Umkristallisieren nicht gereinigt zu werden, sondern können in der Regel als solche, wie sie bei ihrer Herstellung anfallen, d.h. ohne weitere Aufarbeitung verwendet werden.

Vor allem bei separater Zugabe (in beliebiger Reihenfolge) des Leimungsmittels (A) und des Retentionsmittels (B) zur Faserstoffsuspension beim erfindungsgemässen Verfahren zum Leimen von Papier oder Karton ist es zweckmässig, das Leimungsmittel teilweise in Salzform einzusetzen. Solche Salze können bei Bedarf dadurch erhalten werden, dass man nach beendeter Umsetzung der Komponenten (a) und (b) die erhaltenen Umsetzungsprodukte durch Zugabe u.a. eines Alkylamins oder Alkanolamins mit insgesamt höchstens 6 Kohlenstoffatomen, z.B. Trimethylamin, Triäthylamin, Monoethanolamin, Diäthanolamin, vor allem durch Zugabe von Ammoniak oder eines Alkalimetallhydroxydes, beispielsweise Kalium- oder vor allem Natriumhydroxyd, in der Regel in wässrigem Medium bei Raumtemperatur (etwa 15 bis etwa 25°C) in die entsprechenden Salze gegebenenfalls mindestens teilweise überführt. Zweckmässigerweise wird ein Alkalimetallhydroxyd, z.B. Kalium- oder vor allem Natriumhydroxyd oder insbesondere Ammoniak in der Regel in Form ihrer verdünnten, etwa 1 bis 10 gewichtsprozentigen, wässrigen Lösungen verwendet. Zweckmässig wird in der Regel höchstens 2 Mol, vor allem 0,1 bis 1,5, insbesondere 0,9 bis 1,1 Mol Ammoniak oder Alkalihydroxyd pro vorhandene acide Sulfonylcarbonylimidgruppe des Leimungsmittels einsetzt. Die als Salze vorliegenden Leimungsmittel weisen somit acide $-SO_2-NH-CO-$ Gruppen auf, die mindestens teilweise in die Gruppe $-SO_2-\overset{\ominus}{N}-CO-\ M^{\oplus}$ überführt werden, worin $M^{\oplus}$ die entsprechenden Amin-, Ammonium- oder Alkalimetall-Kationen

bedeutet.

Bevorzugte Leimungsmittel (A) der angegebenen Art weisen Molekulargewichte von etwa 200 bis etwa 3000, vorzugsweise etwa 600 bis etwa 2000 und infolge ihres Gehaltes an mindestens einer aciden Sulfonylcarbonylimidgruppe eine Säurezahl (mg KOH/g Substanz) von etwa 15 bis etwa 200, vorzugsweise etwa 25 bis etwa 100 auf.

Im erfindungsgemässen Papierleimungsverfahren wird neben dem neuen, vorstehend beschriebenen, anionischen oder aciden Leimungsmittel (A) stets ein polymeres, kationisches Retentionsmittel (B) eingesetzt, welches in der Regel ein Molekulargewicht von mindestens etwa 1000, vorzugsweise etwa 2000 bis etwa 2'000'000 aufweist. Retentionsmittel mit Molekulargewichten im Bereich von 10'000 bis 100'000 sind besonders bevorzugt. Grundsätzlich kommt jedes handelsübliche Retentionsmittel in Betracht für seinen Einsatz im erfindungsgemässen Verfahren. Als Beispiele herkömmlicher Retentionsmittel (B), die sich besonders gut dazu eignen, zusammen mit dem Leimungsmittel (A) im erfindungsgemässen Papierleimungsverfahren eingesetzt zu werden, seien Polyalkylenimine; Epihalogenhydrin-Addukte von Umsetzungsprodukten aus Polyalkylenpolyaminen und aliphatischen Dicarbonsäuren; Epihalogenhydrin-Addukte von Umsetzungsprodukten aus Polyalkylenpolyaminen, Dicyandiamid und gegebenenfalls unveresterten oder mit Alkanolen veresterten, organischen Dicarbonsäuren; Umsetzungsprodukte aus Dicyandiamid, Formaldehyd, Ammoniumsalzen starker anorganischer Säuren und Alkylendiaminen oder Polyalkylenpolyaminen; kationisch modifizierte Stärken oder Kohlenhydrate aus Johannisbrot- oder Guarkernmehl; Copolymerisate auf Basis von Polyamid-Aminen und Umsetzungsprodukte aus Epihalogenhydrinen und polymerisierten Diallylaminen erwähnt.

Bevorzugte Epichlorhydrin-Addukte von Umsetzungsprodukten aus Polyalkylenpolyaminen und aliphatischen Dicarbonsäuren sind z.B. in der britischen Patentschrift 865 727, Epichlorhydrin-Addukte aus Umsetzungsprodukten aus Dicyandiamid und Diäthylentriamin oder Triäthylentetramin

z.B. in der deutschen Offenlegungsschrift 2 710 061 und in der britischen Patentschrift 1 125 486, Epichlorhydrin-Addukte von Umsetzungsprodukten aus Diäthylentriamin, Dicyandiamid und unveresterten oder vorzugsweise mit Niederalkanolen veresterten Dicarbonsäuren, insbesondere Dimethyladipat, z.B. in der britischen Patentschrift 1 125 486 und Umsetzungsprodukte aus Dicyandiamid, Formaldehyd, Ammoniumsalzen starker anorganischer Säuren und aus Aethylendiamin oder Triäthylentetramin, z.B. in der US Patentschrift 3 491 064 beschrieben. Bevorzugte kationisch modifizierte Stärken oder Kohlenhydrate aus Johannisbrot- oder Guarkernmehl sind Alkylenoxyd-Addukte dieser Stärken oder Kohlenhydrate, wobei das eingesetzte Alkylenoxyd 2 oder 3 Kohlenstoffatome im Alkylenrest und quaternäre Ammoniumgruppen aufweist. Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyäthylenpolyamin, insbesondere Dimethylaminohydroxypropyl-diäthylentriamin, erhältlich. Sie sind z.B. in CTFA Cosmetic Ingredient Dictionary, 3. Auflage 1982, der Cosmetic Toiletry and Fragrance Association beschrieben. Umsetzungsprodukte aus Epihalogenhydrinen und polymerisierten Diallylaminen weisen bevorzugt Molekulargewichte von 1000 bis 2000 auf und sind z.B. in den US-Patentschriften 3 700 623 und 4 279 794 beschrieben.

Als Retentionsmittel (B), die zur Verwendung zusammen mit den Leimungsmitteln (A) im erfindungsgemässen Papierleimungsverfahren im Vordergrund des Interesses stehen, sei eine mit einem quaternäre Ammoniumgruppen enthaltenden Propylenoxyd modifizierte Mais- oder Kartoffelstärke, deren 25 %ige Anschlämmung in destilliertem Wasser bei 20°C einen pH-Wert von 4,2 bis 4,6 aufweist, ein Polyäthylenimin, das ein Molekulargewicht von 10 000 bis 100 000 aufweist, ein Epichlorhydrin-Addukt eines Umsetzungsproduktes aus Triäthylentetramin und Dicyandiamid, ein Epichlorhydrin-Addukt eines Umsetzungsproduktes aus Diäthylentriamin, Dicyandiamid und Dimethyladipat, ein Umsetzungsprodukt aus Dicyandiamid, Formaldehyd, Ammoniumchlorid und Aethylendiamin, ein Epichlorhydrin-Addukt eines Poly-N-methyldiallylamins und ein Copolymerisat aus Adipinsäure und Dimethylamino-hydroxypropyl-diäthylentriamin genannt.

Verfahrengemäss werden in der Regel 0,02 bis 3, vorzugsweise 0,05 bis 3 oder 0,1 bis 3, insbesondere 0,1 bis 0,8 oder 0,2 bis 0,8 Gewichtsprozent des Leimungsmittels (A) und 0,02 bis 3, vorzugsweise 0,05 bis 3 oder 0,1 bis 3, insbesondere 0,1 bis 0,4 oder 0,2 bis 0,4 Gewichtsprozent des Retentionsmittels (B), bezogen jeweils auf Trokkensubstanz an (A) und (B) und auf den Feststoffgehalt der Faserstoffsuspension, eingesetzt. 0,02 bis weniger als 0,05 Gewichtsprozent des Leimungsmittels (A) und des Retentionsmittels (B) reichen nur für das sogenannte "size press control", das mit konventionellen Leimungstests nicht erfassbar ist (vgl. z.B. Artikel "Control and understanding of size press pickup" von D.R. Dill in der Zeitschrift TAPPI (Proceedings of the Technical Association of the Pulp and Paper Industry, Band 57, Nr. 1 von Januar 1974, Seiten 97 bis 100).
Die Faserstoffsuspension, zu welcher die Leimungsmittel (A) und die Retentionsmittel (B) gegeben werden, weist in der Regel einen Feststoffgehalt von 0,1 bis 5, vorzugsweise 0,3 bis 3, insbesondere 0,3 bis 1 Gewichtsprozent und einen Schopper-Riegler-Mahlgrad von etwa 10° bis etwa 60°, vor allem 20 bis 60°, vorzugsweise 20 bis 45°, insbesondere 25 bis 35° auf. Sie enthält in der Regel Zellstoff, insbesondere solchen aus Nadelholz, z.B. Kiefernholz, oder aus Hartholz, d.h. Laubholz, z.B. Buchenholz, der nach herkömmlichen Verfahren, z.B. dem Sulfit- oder vor allem dem Sulfatverfahren hergestellt wird. Zudem enthält die Faserstoffsuspension gegebenenfalls Holzschliff. Auch alaunhaltiges Altpapier kann in der Faserstoffsuspension enthalten sein. Auch Zellstoffsuspensionen, die nach dem sogenannten CMP- oder CTMP-Verfahren (Chemimechanical und chemithermomechanical pulping processes, vgl. z.B. Artikel "Developments in refiner mechanical

pulping" von S.A. Collicutt und Mitarbeitern in TAPPI, Band 64, Nr. 6 von Juni 1981, Seiten 57 bis 61) hergestellt werden, kommen in Betracht.

Die Faserstoffsuspension kann zudem organische oder mineralische Füllmittel enthalten. Als organische Füllmittel kommen u.a. synthetische Pigmente, z.B. Polykondensationsprodukte aus Harnstoff oder Melamin und Formaldehyd mit grossen spezifischen Oberflächen, die in hochdispetser Form vorliegen und z.B. in den britischen Patentschriften 1 043 937 und 1 318 244 beschrieben sind, als mineralische Füllmittel u.a. Montmorillonit, Titandioxyd, Calciumsulfat und vor allem Talk, Kaolin und/oder Kreide (Calciumcarbonat) in Betracht. In der Regel enthält die Faserstoffsuspension 0 bis 40, vorzugsweise 5 bis 25, insbesondere 15 bis 20 Gewichtsprozent, bezogen auf den Feststoffgehalt der Faserstoffsuspension, an Trockensubstanz der Füllmittel der angegebenen Art.

Der pH-Wert der Faserstoffsuspension kann in einem weiten Bereich liegen, wobei z.B. Werte von 3,5 bis etwa 10 vorliegen können.

Bei Zusatz von z.B. Calciumcarbonat werden alkalische Faserstoffsuspensionen mit einem pH-Wert von etwa 7 bis etwa 9, vorzugsweise 7,5 bis 8,5 erhalten. Saure Faserstoffsuspensionen mit einem pH-Wert von 3,5 bis 7, vorzugsweise 5 bis 7, insbesondere 5 bis 6, können in Abwesenheit von Calciumcarbonat durch Zugabe von Säuren, z.B. Schwefel- oder Ameisensäure oder vor allem von z.B. latent sauren Sulfaten, wie Aluminiumsulfat (Alaun), erhalten werden.

Faserstoffsuspensionen,die kein Füllmittel enthalten, können in einem breiten pH-Bereich von z.B. 3,5 bis 10 vorliegen. Bevorzugt sind Faserstoffsuspensionen, die gegebenenfalls durch Zusatz von Kreide einen pH-Wert von etwa 7 bis etwa 9 aufweisen und insofern vorteilhaft sind, dass mögliche Korrosionserscheinungen an den empfindlichen Papiermaschinen ausgeschlossen werden.

Die Faserstoffsuspension kann auch Additive enthalten, wie z.B. Stärke oder ihre Abbauprodukte, welche die Faser/Faser- oder Faser/Füllmittel-Bindung erhöhen.

Auch hochmolekulare Polymere der Acrylsäurereihe, z.B. Polyacrylamide, mit Molekulargewichten über 1'000'000 können zu den Faserstoffsuspensionen als Hilfsmittel zum Zurückhalten feinster Zellstoffaserteilchen gegeben werden, wobei minimale Einsatzmengen von etwa 0,005 bis 0,02 Gewichtsprozent, bezogen auf Trockensubstanz des Polymers und den Feststoffgehalt der Faserstoffsuspensionen, genügend sind.

Die Faserstoffsuspension wird im erfindungsgemässen Verfahren auf an sich bekannte Weise auf Blattbildnern oder vorzugsweise kontinuierlich auf Papiermaschinen üblicher Bauart zu Papier oder Karton weiterverarbeitet. Nach einer Trocknung bei etwa 100 bis 140°C während etwa 0,5 bis 10 Minuten werden Papiere eines variablen Flächengewichtes von z.B. 50 bis 200 $g/m^2$ erhalten.

Wie eingangs erwähnt, enthält die wässrige Zusammensetzung zur Durchführung des erfindungsgemässen Papierleimungsverfahrens neben fakultativen üblichen Zusätzen das Leimungsmittel (A), sofern das Leimungsmittel und das Retentionsmittel (B) separat zur Faserstoffsuspension gegeben werden. In diesem Fall enthält die Zubereitung das Leimungsmittel in der Regel teilweise in Form seiner Salze (erhalten unter Mitverwendung von z.B. Ammoniak, eines Alkyl- oder Alkanolamins oder eines Alkalimetallhydroxydes der angegebenen Art in den vorstehend angegebenen Verhältnissen). Im allgemeinen enthalten solche Zusammensetzungen 5 bis 30, vorzugsweise 5 bis 20 Gewichtsprozent an Trockensubstanz des teilweise in Salzform vorliegenden Leimungsmittels, bezogen auf das Gewicht der wässrigen Zusammensetzung.

Hingegen enthält die wässrige Zusammensetzung neben den fakultativen, üblichen Zusätzen, sofern das Leimungsmittel (A) und das Retentionsmittel (B) gleichzeitig zur Faserstoffsuspension gegeben werden,

(A) 2 bis 40, vorzugsweise 5 bis 30, insbesondere 5 bis 10 Gewichtsprozent Leimungsmittel (berechnet als Trockensubstanz), bezogen auf das Gewicht der wässrigen Zusammensetzung, wobei das Leimungsmittel gegebenenfalls in Salzform vorliegt, und

(B) 0,1 bis 20, vorzugsweise 0,5 bis 10, insbesondere 3 bis 8 Gewichtsprozent Retentionsmittel (berechnet als Trockensubstanz), bezogen auf die wässrige Zusammensetzung.

Die wässrigen Zusammensetzungen der angegebenen Art enthalten gegebenenfalls als übliche Zusätze oberflächenaktive Verbindungen, z.B. Dispergatoren oder ferner Emulgatoren und/oder wasserlösliche, organische Lösungsmittel. Als Dispergatoren und Emulgatoren kommen z.B. herkömmliche Ligninsulfonate, Aethylenoxydaddukte von Alkylphenolen, Fettaminen, Fettalkoholen oder Fettsäuren, Fettsäureester mehrwertiger Alkohole, substituierte Benzimidazole oder Kondensationsprodukte aus aromatischen Sulfonsäuren und Formaldehyd in Betracht. Weitere oberflächenaktive Verbindungen sind vorzugsweise die anionischen Tenside, insbesondere Sulfattenside, z.B. Diäthanolaminlaurylsulfat oder äthoxylierte Laurylsulfate. Mögliche wasserlösliche, organische Lösungsmittel sind aliphatische Aether mit 1 bis 10 Kohlenstoffatomen, z.B. Dioxan, Aethylenglykol-n-butyläther oder Diäthylenglykolmonobutyläther oder Alkohole mit 1 bis 4 Kohlenstoffatomen, z.B. Isopropanol, Aethanol oder Methanol.

Die Zusammensetzungen werden auf übliche Weise hergestellt, indem man das Leimungsmittel (A) zusammen mit dem Retentionsmittel (B) oder das Leimungsmittel (A) in der Regel teilweise in Form seines

Salzes für sich allein entweder in geschmolzenem Zustand oder vorzugsweise in festem Zustand, insbesondere in pulverisierter Form, in der Regel in Gegenwart von Glasperlen und nötigenfalls von Emulgatoren (bei Leimungsmittel in geschmolzenem Zustand) oder Dispergatoren (bei Leimungsmittel in Pulverform) bei höchstens 90°C, vorzugsweise etwa 50 bis etwa 85°C bei Emulsionen, insbesondere bei etwa 15 bis etwa 25°C bei Dispersionen, verrührt, wobei lagerstabile, homogene, weiterverdünnbare Emulsionen oder vorzugsweise Dispersionen erhalten werden. Da die Leimungsmittel zusammen mit den Retentionsmitteln oder die teilweise als Salze vorliegenden Leimungsmittel in der Regel selbst-dispergierend oder selbst-emulgierend sind, ist der Einsatz von Dispergatoren oder Emulgatoren im allgemeinen nicht unbedingt erforderlich. Dies gilt auch für den fakultativen Zusatz von Lösungsmitteln und/oder Tensiden, die nur bei ungenügender Lagerstabilität der Dispersionen oder Emulsionen eingesetzt werden.

Als Vorteil des erfindungsgemässen Verfahrens sei erwähnt, dass Faserstoffsuspensionen der verschiedensten Art mit relativ besonders kleinen Mengen an Leimungs- und Retentionsmittel auf einfache Art und Weise zu Papier verarbeitet werden können, welches gute Leimungseigenschaften (Tintenschwimmdauer und vor allem Wasseraufnahme nach Cobb) aufweist. Das verfahrensgemäss geleimte Papier weist gute mechanische Eigenschaften d.h. gute Festigkeiten, insbesondere eine gute Reissfestigkeit auf. Eine gute Reproduzierbarkeit des Verfahrens ist gewährleistet. Insbesondere können holzschliffhaltige oder altpapierhaltige Faserstoffsuspensionen verarbeitet werden. Auch die Kompatibilität des erfindungsgemäss verwendeten Leimungsmittels mit verschiedenen Füllmitteln wie z.B. Kaolin und auch verschiedenen anderen Zusätzen, wie z.B. Alaun in saurem Bereich der Faserstoffsuspensionen, ist vorteilhaft. Die Leimungsmittel weisen ebenfalls eine vorteilhafte Kompatibilität mit optischen Aufhellern auf. Zudem wird der Weissgrad des geleimten Papiers durch die Leimung nicht wesentlich beeinflusst und kann sogar u.U. verbessert werden. Vor allem ist die in der Regel überraschend hohe Lagerstabilität der Leimungsmitteldispersionen der angegebenen Art von grossem Vorteil.

Die in den nachfolgenden Ausführungsbeispielen angegebenen Teile und Prozente beziehen sich auf das Gewicht.

Herstellung neuer Verbindungen als Leimungsmittel

Beispiel 1: 270 Teile Octadecanol (1 Mol) werden in 1200 Teilen Toluol gelöst. Zu dieser Lösung wird eine Lösung von 112 Teilen 4-Isocyanato-benzolsulfonylisocyanat (0,5 Mol) in 500 Teilen Toluol innerhalb von einer Stunde gegeben, wobei die Temperatur des Reaktionsgemisches durch Aussenkühlung zwischen 20 und 25°C gehalten wird. Das Reaktionsgemisch wird nach beendeter Zugabe auf 50°C aufgeheizt und während einer Stunde bei dieser Temperatur unter Rühren gehalten. Anschliessend wird das Toluol unter vermindertem Druck aus dem Reaktionsgemisch abdestilliert.

Man erhält als weisses Pulver 365 Teile des Umsetzungsproduktes der Formel (17).

Zu Analysenzwecken wird ein Teil des Umsetzungsproduktes aus Dioxan umkristallisiert. Das umkristallisierte Produkt weist einen Schmelzpunkt von 125-127°C und eine Säurezahl von 74 auf.

Beispiel 2: Man verfährt wie im Beispiel 1 angegeben, setzt jedoch 269 Teile Octadecylamin (1 Mol) (anstelle von 270 Teilen Octadecanol) ein. Man erhält als weisses Pulver 362 Teile des Umsetzungsproduktes der Formel (18), das nach dem Umkristallisieren einen Schmelzpunkt von 159-166°C und eine Säurezahl von 72 aufweist.

Beispiel 3: Man verfährt wie im Beispiel 1 angegeben, setzt jedoch 522 Teile Dioctadecylamin (1 Mol) (anstelle von 270 Teilen Octadecanol)

ein. Man erhält als weisses Pulver 603 Teile des Umsetzungsproduktes der Formel (19), das nach dem Umkristallisieren einen Schmelzpunkt von 92-95°C und eine Säurezahl von 43 aufweist.

Beispiel 4: Man verfährt wie im Beispiel 1 angegeben, setzt jedoch 631 Teile eines Kondensationsproduktes aus 2 Mol Stearinsäure und 1 Mol Diäthylentriamin (1 Mol) (anstelle von 270 Teilen Octadecanol) ein. Man erhält als ockerfarbenes Pulver 714 Teile des Umsetzungsproduktes der wahrscheinlichen Formel (20), das als Rohprodukt einen Schmelzpunkt von 90-100°C und eine Säurezahl von 39 aufweist.

Beispiel 5: Man verfährt wie im Beispiel 1 angegeben, setzt jedoch 112 Teile 3-Isocyanato-benzolsulfonyl-isocyanat (0,5 Mol) (anstelle von 112 Teilen 4-Isocyanato-benzolsulfonyl-isocyanat) ein und kristallisiert ein Teil des Rohproduktes aus Aceton um. Man erhält als weisses Pulver 367 Teile des Umsetzungsproduktes der Formel (21), das nach dem Umkristallisieren einen Schmelzpunkt von 90-92°C und eine Säurezahl von 73 aufweist.

Beispiel 6: Man verfährt wie im Beispiel 1 angegeben, setzt jedoch 72 Teile 2-Isocyanato-äthan-1-sulfonylisocyanat (0,5 Mol) (anstelle von 112 Teilen 4-Isocyanato-benzolsulfonyl-isocyanat) ein und kristallisiert ein Teil des Rohproduktes aus Essigsäureäthylester um. Man erhält als weisses Pulver 326 Teile des Umsetzungsproduktes der Formel (22), das nach dem Umkristallisieren einen Schmelzpunkt von 53-54,5°C und eine Säurezahl von 78 aufweist.

## Applikationsbeispiele

Beispiele 7 bis 13: Eine Faserstoffsuspension aus gebleichtem Birkensulfatzellstoff und Kiefernsulfatzellstoff im Gewichtsverhältnis 1:1 in Wasser von 10° dH (deutsche Härtegrade), die einen Schopper-Riegler

Mahlgrad von 35° und einen Feststoffgehalt von 0,5% aufweist, wird mit 20% Kreide als Füllmittel und hierauf mit 0,01% PERCOL 292® (kationisches, hochmolekulares (MG (>1·10^7) Polyacrylamid) als Hilfsmittel zum Zurückhalten feinster Zellstoffaserteilchen versetzt, wobei sich der in der nachfolgenden Tabelle I angegebene pH-Wert der Faserstoffsuspension einstellt. Die Prozentangaben beziehen sich auf Trockensubstanz an Hilfs- und Füllmittel, bezogen auf den Feststoffgehalt der Faserstoffsuspension.

Formulierungen des Leimungsmittels werden hergestellt, indem jeweils 7% der angegebenen Leimungsmittel in Pulverform wie sie bei der Herstellung anfallen mit jeweils 3,5% POLYMIN P® (Polyäthylenimin eines Molekulargewichts von 10'000 bis 100'000) als Retentionsmittel in Gegenwart von entionisiertem Wasser und von Glasperlen mit einem Durchmesser von 2 mm bei Raumtemperatur (15 bis 25°C) verrührt werden. Die erhaltenen Dispersionen sind giessbar, homogen und lagerstabil. Die Prozentangaben beziehen sich auf die Trockensubstanz an Leimungs- und Retentionsmittel, bezogen auf das Gesamtgewicht der Formulierung.

Nun wird die wässrige Formulierung des Leimungsmittels und des Retentionsmittels zur Faserstoffsuspension so zugegeben, dass die in der nachfolgenden Tabelle I angegebenen Einsatzmengen an Trockensubstanz des Leimungsmittels, bezogen auf den Feststoffgehalt der Faserstoffsuspension, entsteht. Anschliessend wird die Faserstoffsuspension in einem Labor-Blattbildner "Formette Dynamique" der Fa. Allimand, Grenoble, Frankreich, zu Papierblättern verarbeitet, die nach der Trocknung bei 130°C während 3 Minuten ein Flächengewicht von 80 $g/m^2$ aufweisen.

Beide Oberflächen der erhaltenen Papierblätter, d.h. die auf der Siebseite des Blattbildners erhaltene Oberfläche und die Gegen- oder Oberseite werden auf ihre Leimungseigenschaften geprüft. Zu diesem Zweck wird die Wasseraufnahme nach Cobb bei 30 Sekunden Einwirkungsdauer (WA $Cobb_{30}$) gemäss DIN 53 132 gemessen. Die Ergebnisse der WA $Cobb_{30}$-Messungen in $g/m^2$ der Siebseite (SS) und Oberseite (OS) nach der Trocknung bei 130°C und nach einer Lagerung von einem Tag bei 20°C sind in der nachfolgenden Tabelle I angegeben. Je geringer die Wasseraufnahme, desto besser ist die Leimung des Papiers. WA $Cobb_{30}$ Werte über 100 entsprechen einer völlig unbefriedigenden Leimung des Papiers.

Tabelle I

| Beispiel Nr. | Leimungsmittel | Einsatzmenge (%) | pH-Wert der Suspension | WA $Cobb_{30}$ ($g/m^2$) | | | |
|---|---|---|---|---|---|---|---|
| | | | | nach Trocknung | | nach 1 Tag Lagerung | |
| | | | | SS | OS | SS | OS |
| 7 | Umsetzungsprodukt gemäss Beispiel 1 | 0,5 | 8,7 | 18 | 13 | 15 | 10 |
| 8 | Umsetzungsprodukt gemäss Beispiel 1 | 0,25 | 8,7 | 16 | 13 | 13 | 11 |
| 9 | Umsetzungsprodukt gemäss Beispiel 2 | 0,2 | 8,7 | 17 | 13 | 16 | 12 |
| 10 | Umsetzungsprodukt gemäss Beispiel 3 | 0,5 | 8,7 | 17 | 13 | 15 | 11 |
| 11 | Umsetzungsprodukt gemäss Beispiel 4 | 0,5 | 8,7 | 19 | 13 | 16 | 11 |
| 12 | Umsetzungsprodukt gemäss Beispiel 6 | 0,5 | 8,7 | 19 | 13 | 16 | 12 |
| 13 | Umsetzungsprodukt gemäss Beispiel 5 | 0,5 | 8,7 | 20 | 14 | 24 | 14 |

Aehnliche Ergebnisse werden erzielt, wenn man an Stelle von POLYMIN P® als Retentionsmittel CATO 110® (kationisch modifizierte Stärke, die mit einem quaternären Ammoniumgruppen enthaltenden Propylenoxyd modifiziert ist und deren pH-Wert der 25%-igen Anschlämmung in destilliertem Wasser bei 20°C 4,2 bis 4,6 beträgt), ein Kondensationsprodukt aus Dicyandiamid und Triäthylentetramin, das mit Epichlorhydrin weiter umgesetzt und gemäss Beispiel 2 der deutschen Offenlegungsschrift 2 710 061 hergestellt wird, ein Umsetzungsprodukt aus Dicyandiamid, Formaldehyd, Ammoniumchlorid und Aethylendiamin, das gemäss Beispiel 1 der US-Patentschrift 3 491 064 hergestellt wird, oder RETAMINOL K® (Polyäthylenimin eines Molekulargewichtes von 20'000 bis 40'000) einsetzt. Hingegen wird nur eine schlechte Leimung mit Cobb-Werten von etwa 150 bis etwa 200 erhalten, wenn man ein Leimungsmittel gemäss einem der Beispiele 1 bis 6, jedoch kein Retentionsmittel, oder ein Retentionsmittel der vorstehend angegebenen Art, jedoch kein Leimungsmittel einsetzt.

<u>Beispiel 14 bis 19:</u> Man verfährt wie in Beispielen 7 bis 13 angegeben, gibt jedoch das Leimungsmittel und das Retentionsmittel separat zur Faserstoffsuspension, wobei 6% oder 15% Leimungsmittel in Pulverform bei Raumtemperatur (15 bis 25°C) in Gegenwart von Wasser und Glasperlen mit einer wässrigen 5%igen Ammoniaklösung zu einer selbstemulgierenden, ebenfalls giessbaren, homogenen und lagerstabilen Emulsion verrührt werden und wobei die in der nachfolgenden Tabelle II angegebenen Formulierungen des Leimungsmittels entstehen. Die angegebenen Val% bedeuten die Anzahl Aequivalente an Ammoniak für 100 Aequivalente, bezogen auf die Anzahl vorhandener, acider Sulfonylcarbonylimidgruppen der jeweils eingesetzten Leimungsmittel. 10 Sekunden nach der Zugabe der angegebenen Einsatzmenge an Trockensubstanz des Leimungsmittel wird die Faserstoffsuspension mit jeweils der angegebenen Einsatzmenge an Trockensubstanz POLYMIN P® als Retentionsmittel versetzt, wobei sich die Einsatzmengen an Leimungs- und Retentionsmittel auf den Feststoffgehalt der Faserstoffsuspension beziehen. Die Leimungsergebnisse sind ebenfalls aus der Tabelle II zu entnehmen.

Tabelle II

| Beispiel Nr. | Leimungsmittel Formulierung | Einsatzmenge Leimungsmittel (%) | Einsatzmenge Retentionsmittel (%) | Füllmittel | pH-Wert der Suspension | WA $Cobb_{30}$ ($g/m^2$) | | | |
|---|---|---|---|---|---|---|---|---|---|
| | | | | | | nach Trocknung | | nach 1 Tag Lagerung | |
| | | | | | | SS | OS | OS | SS |
| 14 | 6% Umsetzungsprodukt gemäss Beispiel 1 100 Val% Ammoniak | 0,3 | 0,25 | 20% Kreide | 8,7 | 17 | 14 | 15 | 14 |
| 15 | 15% Umsetzungsprodukt gemäss Beispiel 2 100 Val% Ammoniak | 0,3 | 0,25 | 20% Kreide | 8,6 | 24 | 15 | 20 | 13 |
| 16 | 15% Umsetzungsprodukt gemäss Beispiel 2 100 Val% Ammoniak | 0,4 | 0,25 | 20% Kreide | 8,7 | 20 | 14 | 18 | 12 |
| 17 | 15% Umsetzungsprodukt gemäss Beispiel 4 100 Val% Ammoniak | 0,35 | 0,25 | 20% Kreide | 8,7 | 61 | 26 | 53 | 18 |
| 18 | 6% Umsetzungsprodukt gemäss Beispiel 5 100 Val% Ammoniak | 0,5 | 0,25 | 20% Kreide | 8,7 | 14 | 13 | 14 | 13 |
| 19 | 15% Umsetzungsprodukt gemäss Beispiel 6 100 Val% Ammoniak | 0,5 | 0,25 | 20% Kreide | 8,7 | 17 | 14 | 38 | 26 |

Bei Verwendung von 10 bis 200 Val% Ammoniak oder Natriumhydroxyd (als 5%ige wässrige Lösungen) zur Formulierung des Leimungsmittels werden ähnlich gute Leimungsergebnisse wie die in Tabelle II angegebenen erhalten.

Aehnliche Ergebnisse werden auch erzielt, wenn man zur Faserstoffsuspension das Retentionsmittel zuerst und 10 Sekunden hierauf das Leimungsmittel gibt. Das gleiche gilt auch, wenn auf die Zugabe von PERCOL 292® und/oder auf die Zugabe eines Füllmittels verzichtet wird. Aehnliche Ergebnisse werden ebenfalls erzielt, wenn man an Stelle von Kreide als Füllmittel Talk oder Kaolin oder wenn man Kreide in Gegenwart von Alaun einsetzt. Auch bei Einsatz von holzschliffhaltigen Faserstoffsuspensionen werden gute Leimungsergebnisse erhalten.

Patentansprüche

1. Verfahren zum Leimen von Papier oder Karton, dadurch gekennzeichnet, dass man zu wässrigen, cellulosehaltigen, gegebenenfalls füllmittelhaltigen Faserstoffsuspensionen mindestens
(A) ein Leimungsmittel, welches 1 oder 2 anionische oder acide, gegebenenfalls in Salzform vorliegende Sulfonylcarbonylimidgruppen ($-SO_2-NH-CO-$) und 2 bis 6 hydrophobe Substituenten mit jeweils mindestens 5 Kohlenstoffatomen enthält, wobei mindestens einer der hydrophoben Substituenten mindestens 8 Kohlenstoffatome aufweist und mindestens zwei der hydrophoben Substituenten miteinander über ein Brückenglied verknüpft sind, das der Formel

$$-CO-NH-A_1-(SO_2-NH-CO)_t-$$

entspricht, worin $A_1$ einen zwei- oder dreiwertigen aliphatischen, cycloaliphatischen oder aromatischen Rest mit höchstens 10 Kohlenstoffatomen und t 1 oder 2 bedeuten, und
(B) ein polymeres, kationisches Retentionsmittel in beliebiger Reihenfolge oder gleichzeitig hinzugibt.

2. Verfahren nach Anspruch 1, dadurch gekennzeichnet, dass man als Komponente (A) ein Leimungsmittel einsetzt, das 1 acide Sulfonylcarbonylimidgruppe und 2 bis 4 hydrophobe Substituenten mit jeweils 8 bis 22 Kohlenstoffatomen enthält, wobei mindestens zwei der hydrophoben Substituenten über ein Brückenglied der Formel

$$-CO-NH-A_2-SO_2-NH-CO-,$$

worin $A_2$ Cycloalkylen, Naphthylen, unsubstituiertes oder durch Methyl substituiertes Phenylen oder Alkylen mit 2 bis 6 Kohlenstoffatomen bedeutet, verknüpft sind.

3. Verfahren nach einem der Ansprüche 1 oder 2 dadurch gekennzeichnet, dass man als Komponente (A) eine Leimungsmittel einsetzt, welches durch Umsetzung von

(a) 1 Mol einer Verbindung der Formel

$$O=C=N-A_1-(SO_2-N=C=O)_t \quad ,$$

worin $A_1$ und t die in Anspruch 1 angegebenen Bedeutungen haben, mit

(b) t+1 Mol eines Fettalkohols, eines primären oder sekundären Fettamins und/oder eines Kondensationsproduktes aus einer Fettsäure und einem Alkylendiamin oder Polyalkylenpolyamin, wobei das Kondensationsprodukt mindestens eine freie Aminogruppe aufweist,
erhältlich ist.

4. Verfahren nach Anspruch 3, dadurch gekennzeichnet, dass man als Komponente (b) einen Fettalkohol mit 8 bis 22 Kohlenstoffatomen, ein Mono- oder Dialkylamin, ein Mono- oder Dialkenylamin mit je 8 bis 22 Kohlenstoffatomen im Alkyl oder Alkenylrest und/oder ein Kondensationsprodukt aus m Mol einer Fettsäure mit 8 bis 22 Kohlenstoffatomen und 1 Mol eines Alkylendiamins oder Polyalkylenpolyamins der Formel

$$H_2N-D_1-(NH-D_2)_{n-1}-NH_2$$

einsetzt, worin $D_1$ und $D_2$ je Propylen oder Aethylen, n eine ganze Zahl von 1 bis 5 und m eine ganze Zahl von 1 bis n bedeuten.

5. Verfahren nach einem der Ansprüche 1 bis 4, dadurch gekennzeichnet, dass man als Komponente (A) ein Leimungsmittel der Formel

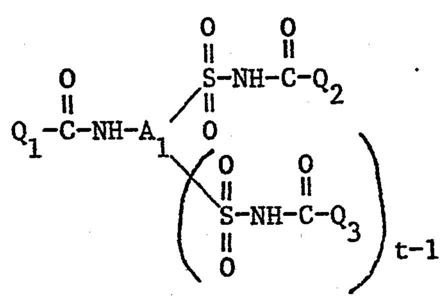

einsetzt, worin $A_1$ einen zwei- oder dreiwertigen aliphatischen cycloaliphatischen oder aromatischen Rest mit höchstens 10 Kohlenstoff-

atomen, $Q_1$, $Q_2$ und $Q_3$

je $-O-R_1$, $-NH-R_1$, 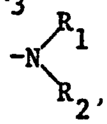 oder

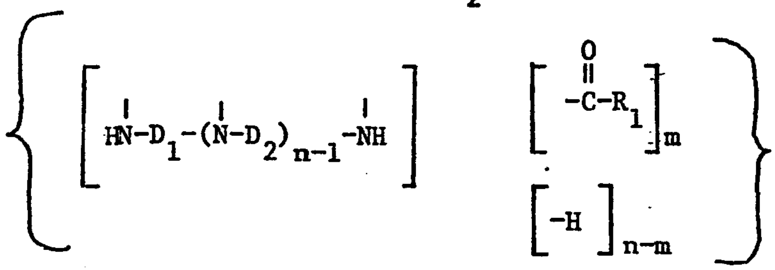 ,

$D_1$ und $D_2$ je Aethylen oder Propylen, $R_1$ und $R_2$ je Alkyl oder Alkenyl mit 8 bis 22 Kohlenstoffatomen, n eine ganze Zahl von 1 bis 5, t 1 oder 2 und m eine ganze Zahl von 1 bis n bedeuten.

6. Verfahren nach Anspruch 5, dadurch gekennzeichnet, dass man als Komponente (A) ein Leimungsmittel der Formel

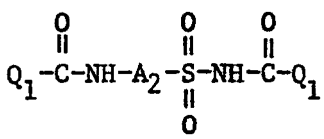 einsetzt,

worin $A_2$ Cycloalkylen, Naphthylen, unsubstituiertes oder durch Methyl substituiertes Phenylen oder Alkylen mit 2 bis 8 Kohlenstoffatomen,

$Q_1$ $-OR_1$, $-NH-R_1$, 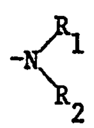 oder

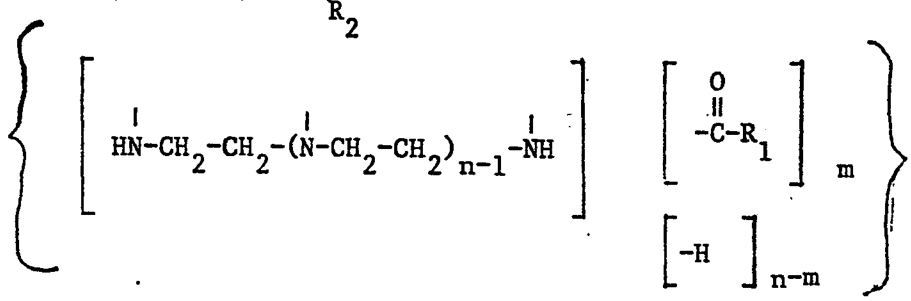 ,

$R_1$ Alkyl oder Alkenyl mit 8 bis 22 Kohlenstoffatomen, n eine ganze Zahl von 1 bis 5 und m eine ganze Zahl von 1 bis n bedeuten.

7. Verfahren nach einem der Ansprüche 1 bis 6, dadurch gekennzeichnet, dass man als Retentionsmittel (B) ein Polyalkylenimin, Epihalogenhydrin-Addukte von Umsetzungsprodukten aus Polyalkylenpolyaminen und aliphatischen Dicarbonsäuren; Epihalogenhydrin-Addukte aus Polyalkylenpolyaminen,

Dicyandiamid und gegebenenfalls unveresterten oder mit Alkanolen veresterten, organischen Dicarbonsäuren; Umsetzungsprodukte aus Dicyandiamid, Formaldehyd, Ammoniumsalzen starker anorganischer Säuren und Alkylendiaminen oder Polyalkylendiaminen; kationisch modifizierte Stärken oder Kohlenhydrate aus Johannisbrot- oder Guarkernmehl; Copolymerisate auf Basis von Polyamid-Aminen oder Umsetzungsprodukte aus Epihalogenhydrinen und polymerisierten Diallylaminen einsetzt.

8. Wässrige Zusammensetzung zur Durchführung des Verfahrens gemäss einem der Ansprüche 1 bis 7, dadurch gekennzeichnet, dass sie das mindestens teilweise in Form von Salzen vorliegende Leimungsmittel (A) und gegebenenfalls übliche Zusätze enthält.

9. Wässrige Zusammensetzung zur Durchführung des Verfahrens gemäss einem der Ansprüche 1 bis 7, worin das Leimungsmittel (A) und das Retentionsmittel (B) gleichzeitig zur Faserstoffsuspension gegeben werden, dadurch gekennzeichnet, dass sie
(A) 2 bis 40 Gewichtsprozent Leimungsmittel,
(B) 0,1 bis 20 Gewichtsprozent Retentionsmittel,
bezogen auf Trockensubstanz von (A) und (B) und auf das Gewicht der wässrigen Zusammensetzung, und gegebenenfalls übliche Zusätze enthält.

10. Verbindungen der Formel

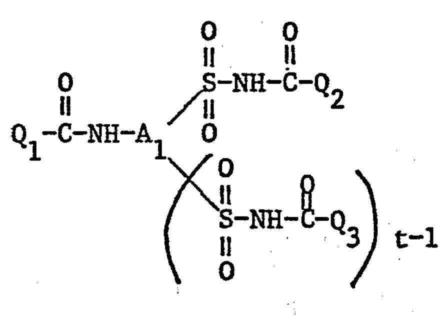

worin $A_1$ einen zwei- oder dreiwertigen aliphatischen, cycloaliphatischen oder aromatischen Rest mit höchstens 10 Kohlenstoffatomen, $Q_1$, $Q_2$ und $Q_3$ je $-O-R_1$, $-NH-R_1$,

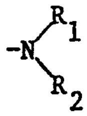 oder

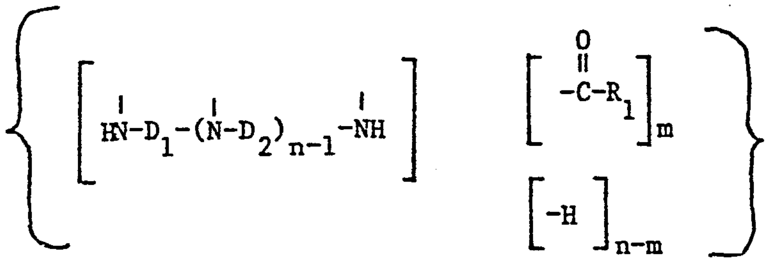 ,

$D_1$ und $D_2$ je Aethylen oder Propylen, $R_1$ und $R_2$ je Alkyl oder Alkenyl mit 8 bis 22 Kohlenstoffatomen, t 1 oder 2, n eine ganze Zahl von 1 bis 5 und m eine ganze Zahl von 1 bis n bedeuten.

11. Verfahren zur Herstellung der Verbindungen gemäss Anspruch 10, dadurch gekennzeichnet, dass man
1 Mol der Verbindung der Formel

$O{=}C{=}N{-}A_1{-}(SO_2{-}N{=}C{=}O)_t$ ,

mit t+1 Mol eines Fettalkohols der Formel

$R_1{-}OH$,

mit t+1 Mol eines primären oder sekundären Fettamins der Formel

$R_1{-}NH_2$ oder $R_1{-}NH{-}R_2$, oder

mit t+1 Mol eines Kondensationsproduktes der Formel

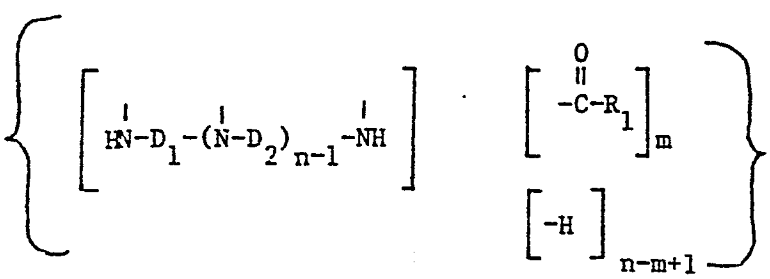 ,

aus m Mol einer Fettsäure und 1 Mol eines Alkylendiamins oder Polyalkylenpolyamins,
wobei $A_1$, $D_1$, $D_2$, $R_1$, $R_2$, m, n und t die in Anspruch 10 angegebenen Bedeutungen haben,
umsetzt.

FO 7.1 PLP/cw*/rz*

0144284

Europäisches Patentamt

# EUROPÄISCHER RECHERCHENBERICHT

Nummer der Anmeldung

EP 84 81 0511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-1 621 698 (MONSANTO)<br>* Anspruch 6 *<br>--- | 1,7-9 | D 21 D 3/00<br>D 21 H 3/02<br>C 07 C 143/83<br>C 07 C 143/833 |
| A | DE-C- 931 887 (DEHYDAG DEUTSCHE HYDRIERWERKE)<br>* Anspruch 2 *<br>--- | 2-4,10<br>11 | |
| A | US-A-3 084 092 (H. ARLT Jr.)<br>--- | | |
| P,X | EP-A-0 096 654 (CIBA-GEIGY)<br>* Ansprüche 1,2,4,19-22 *<br>--- | 1,7-9 | |
| | | | RECHERCHIERTE SACHGEBIETE (Int. Cl.4) |
| E | EP-A-0 123 763 (CIBA-GEIGY)<br>* Ansprüche 1-13 *<br>--- | 1,4,<br>7-9 | C 07 C<br>D 21 D<br>D 21 H |
| X | US-A-3 454 606 (T. BROTHERTON et al.)<br>* Spalte 1, Zeile 17 - Spalte 2, Zeile 37; Spalte 8, Zeile 30 - Spalte 9, Zeile 19 *<br>----- | 10,11 | |

Der vorliegende Recherchenbericht wurde für alle Patentansprüche erstellt.

| Recherchenort | Abschlußdatum der Recherche | Prüfer |
|---|---|---|
| DEN HAAG | 10-01-1985 | NESTBY M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82